# EUROPEAN PATENT APPLICATION

(11) **EP 2 886 148 A1**
(43) Date of publication of application: **24.06.2015**
(21) Application number: 13199020.2
(22) Date of filing: 20.12.2013
(51) Int. Cl.: A61M 5/42

(54) **Drug delivery aid**

(71) Applicant: Sanofi-Aventis Deutschland GmbH, 65929 Frankfurt am Main (DE)
(72) Inventor: The designation of the inventor has not yet been filed

(57) **Abstract**

The invention relates to a drug delivery aid (6), comprising a substantially flat contact element (7) with a first surface (8) adapted to support a drug delivery device (1) with an extendable injection needle (4), and a second surface (12) opposite the first surface (8) adapted to be placed on an injection site, wherein a first aperture (9) is arranged in the contact element (7) adapted to allow extension of the injection needle (4) of the drug delivery device (1) through the first aperture (9).

## Description

### Technical Field

The invention relates to a drug delivery aid for facilitating application of the drug delivery device.

### Background of the Invention

Administering an injection is a process which presents a number of risks and challenges for users and healthcare professionals, both mental and physical. Pre-filled syringes that are filled with a selected dosage of a medicament for administering the medicament to a patient are known in the art. Drug delivery devices comprising a needle cover for covering a needle of a pre-filled syringe before and after use are also known. Typically, the needle cover is either manually moved or moved by the action of a relaxing spring to surround the needle.

There remains a need for an improvement for facilitating application of the drug delivery device.

### Summary of the Invention

It is an object of the present invention to provide an improved drug delivery aid.

The object is achieved by a drug delivery aid according to claim 1.

Exemplary embodiments of the invention are given in the dependent claims.

According to the invention a drug delivery aid comprises a substantially flat contact element with a first surface adapted to support a drug delivery device with an extendable injection needle, and a second surface opposite the first surface adapted to be placed on an injection site, wherein a first aperture is arranged in the contact element adapted to allow extension of the injection needle of the drug delivery device through the first aperture.

As the second surface of the contact element is remarkably greater than the footprint of the needle cover of the drug delivery device, irrespective of its softness the injection site will yield less and the needle cover may be reliably depressed into the housing of the drug delivery device for unlocking the trigger button or triggering the injection depending on the type of drug delivery device applied.

In an exemplary embodiment the contact element may be substantially disk shaped.

In an exemplary embodiment the first surface and the second surface may be substantially circular.

In another exemplary embodiment the first surface and the second surface may be substantially elliptical.

In an exemplary embodiment a recess may be arranged around the first aperture for centering a distal end of a drug delivery device such that the injection needle is axially aligned with the first aperture.

In an exemplary embodiment a handle may be arranged on the contact element.

In an exemplary embodiment one end of the handle may be arranged on the contact element, wherein another end of the handle is attached to a retaining element for releasably attaching the drug delivery aid to a drug delivery device.

In an exemplary embodiment at least the handle and/or the retaining element may be resilient.

In an exemplary embodiment the retaining element may comprise a second aperture for allowing insertion of the drug delivery device through the second aperture.

In an exemplary embodiment an internal diameter of the second aperture may be smaller than an external diameter of the drug delivery device.

In an exemplary embodiment the second surface may comprise a surface structure.

In an exemplary embodiment the surface structure may comprise a plurality of pins.

In an exemplary embodiment the pins may comprise a mushroom shape.

In an exemplary embodiment the drug delivery aid may be part of a drug delivery kit, further comprising at least one drug delivery device.

In an exemplary embodiment the drug delivery device may comprise a housing and a needle cover telescoped in a distal end of the housing and adapted to be depressed towards the housing for triggering an injection or for unlocking a trigger button on the housing, wherein the recess in the drug delivery aid has a diameter corresponding to a diameter of the needle cover.

Further scope of applicability of the present invention will become apparent from the detailed description given hereinafter. However, it should be understood that the detailed description and specific examples, while indicating exemplary embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: is a schematic view of an exemplary embodiment of a drug delivery device,
- Figure 2: is a schematic perspective view of a drug delivery aid with a contact element having a first surface,
- Figure 3: is a schematic perspective view of a second surface of the contact element, and
- Figure 4: is a schematic view of a drug delivery device with another exemplary embodiment of a drug delivery aid.

Corresponding parts are marked with the same reference symbols in all figures.

### Detailed Description

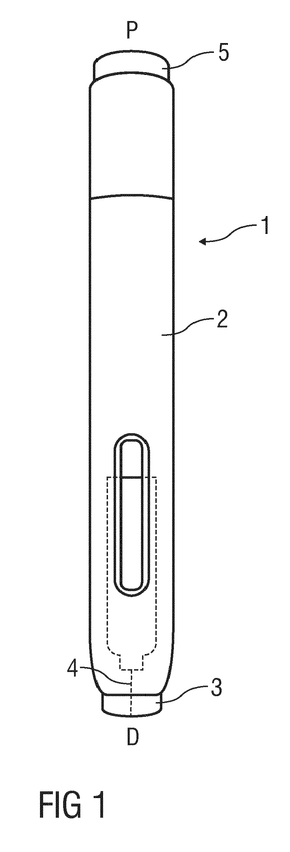

Figure 1 is a schematic view of an exemplary embodiment of a drug delivery device 1 comprising a housing 2 and a needle cover 3 telescoped in end of the housing 2 pointing in a distal direction D. An injection needle 4 is extendible from the drug delivery device 1 through the needle cover 3. The injection needle 4 may be attached or attachable to a drug cartridge arranged within the housing 2. A trigger button 5 is arranged at an end of the housing 2 pointing in a proximal direction P. The needle cover 3 may be biased in the distal direction D towards the position shown in figure 1. In this position the trigger button 5 may be locked so as to prevent it from being operated. In order to initiate an injection, the needle cover 3 has to be depressed in the proximal direction P towards the housing 2, e.g. by pressing it against an injection site such as a patient's skin, thus unlocking the trigger button 5 which can then be depressed for starting the injection.

In an exemplary embodiment the injection needle 4 may be in a fixed axial position relative to the housing 2 in such a manner that the injection needle 4 is covered by the needle cover 3 in the position of figure 1 but exposed when the needle cover 3 is depressed into the housing 2.

In an exemplary embodiment the injection needle may be slidably arranged within the housing 2 and arranged to be advanced by a drive means (not illustrated) of the drug delivery device 1, e.g. a spring, once the injection has been started by depression of the trigger button 5.

In an exemplary embodiment the drug delivery device 1 may not comprise a trigger button. In this case the injection may be triggered immediately by depression of the needle cover 3.

In an exemplary embodiment it may be required to remove a protective needle shield (not illustrated) from the injection needle 4 prior to application of the drug delivery device 1 to the injection site.

If the injection site is soft as may be the case with obese patients sufficient depression of the needle cover 3 may be difficult resulting in the trigger button not being unlocked or the injection not being started.

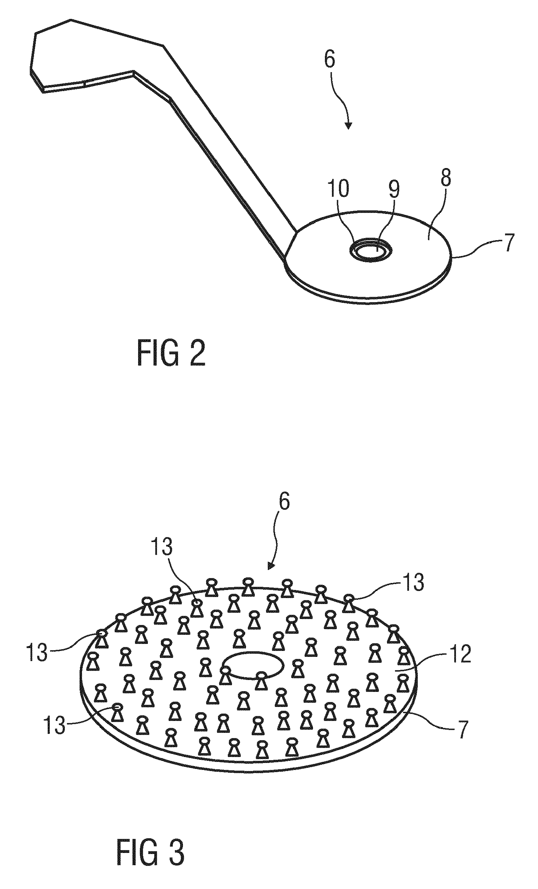

Figure 2 is a schematic perspective view of a drug delivery aid 6, comprising a substantially flat contact element 7 with a first surface 8 adapted to support a drug delivery device 1. A first aperture 9 is arranged in the contact element 7 adapted to allow extension of the injection needle 4 of the drug delivery device 1 through the first aperture 9 when the drug delivery device 1 is applied on the first surface 8. The contact element 7 is substantially disk shaped, in particular such that the first surface 8 is circular.

In another exemplary embodiment the first surface 8 is substantially elliptical or oval. Circular, elliptical, oval or other non-angled shapes may be more convenient for the patient than angled shapes.

A recess 10 is arranged around the first aperture 9 for centering a distal end, e.g. the needle cover 3 of the drug delivery device 1 such that the injection needle 4 is axially aligned with the first aperture 9.In the illustrated embodiment the recess 10 is circular corresponding to the circular cross section of the needle cover 3. The recess 10 may have a diameter corresponding to a diameter of the needle cover 3.

In other exemplary embodiments the recess 10 and the needle cover 3 may have other corresponding shapes, e.g. elliptical, oval, angled or irregular.

A handle 11 is arranged on the contact element 7. In an exemplary embodiment the handle 11 is attached to an edge of the contact element 7. In an exemplary embodiment the contact element 7 and the handle 11 are integrally shaped. The handle 11 allows a user to hold the drug delivery aid 6 with one hand, similar to a spoon, and the drug delivery device 1 with the other. However, the handle 11 is not an essential feature as the drug delivery device 1 may likewise be used with the contact element 7 only. In another exemplary embodiment the drug delivery device 1 and the contact element 7 may be fixed to each other, e.g. by an adhesive or by a snap fit (not illustrated).

Figure 3 is a schematic perspective view of a second surface 12 of the contact element 7.
The second surface 12 is arranged opposite the first surface 8 and adapted to be placed on an injection site. In an exemplary embodiment the second surface 12 comprises a surface structure 13 adapted to stimulate nerves on the skin of a patient when the contact element 7 is applied there. This may mitigate or prevent pain felt by the user when inserting the needle 4 into the skin.

In an exemplary embodiment the surface structure 13 comprises a plurality of pins.

In an exemplary embodiment the pins comprise a mushroom shape.

In other exemplary embodiments the pins may have a different shape or the surface structure 13 may be different, e.g. without pins.

In order to perform an injection, the second surface 12 of the contact element 7 may be placed onto the injection site, e.g. by a user holding the handle 11. The first aperture 9 allows for viewing the spot intended to be pierced by the injection needle 4. The drug delivery device 1 is then applied on the first surface 8 such that the needle cover 3 is positioned within the recess 10 thereby centering the injection needle 4 with respect to the first aperture 9.

As the second surface 12 of the contact element 7 is remarkably greater than the footprint of the needle cover 3 the injection site will yield less and the needle cover 3 may be reliably depressed into the housing 2 of the drug delivery device 1 for unlocking the trigger button 5 or triggering the injection depending on the type of drug delivery device 1 applied.

Due to the recess 10 the injection needle 4 is maintained centered in the first aperture 9 throughout the injection.

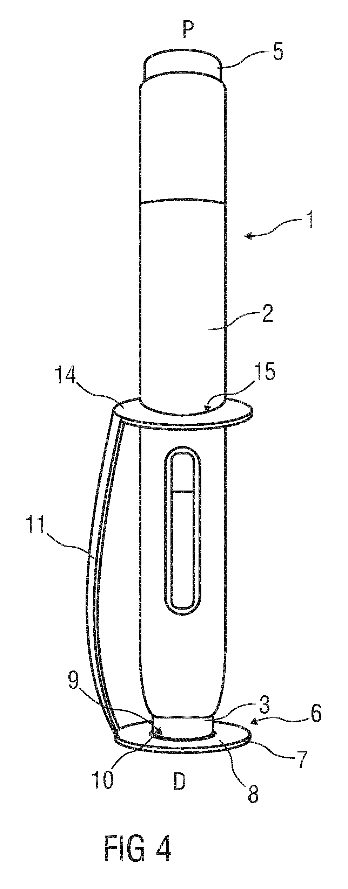

Figure 4 is a schematic view of a drug delivery device 1 with another exemplary embodiment of a drug delivery aid 6. The drug delivery device 1 also comprises a contact element 7 and a handle 11. One end of the handle 11 is arranged on the contact element 7, wherein another end of the handle 11 is attached to a retaining element 14 for releasably attaching the drug delivery aid 6 to the drug delivery device 1.

In an exemplary embodiment at least the handle 11 and/or the retaining element 14 are/is resilient or comprise/comprises a resilient material. Likewise, the contact element 7 may be resilient. The contact element 7, the handle 11 and the retaining element 14 may be integrally shaped. In an exemplary embodiment the handle 11 may be connected to the contact element 7 and to the retaining element 14 through live hinges (not illustrated).

The retaining element 14 comprises a second aperture 15 for allowing insertion of the drug delivery device 1. In an exemplary embodiment an internal diameter of the second aperture 15 is slightly smaller than an external diameter of the drug delivery device 1, in particular of the housing 2 such that the drug delivery device 1 and the drug delivery aid 6 are frictionally engaged. In particular in this embodiment, the retaining element 14 is resilient or comprises a resilient material. In the embodiment of figure 3 the drug delivery device 1 and the contact element 7 may be fixed to each other, e.g. by an adhesive or by a snap fit (not illustrated) thus allowing operation of the drug delivery device 1 with only one hand.

The drug delivery aid 6 may be adapted to be reusable. The drug delivery device 1 may be a disposable or a reusable device.

The drug delivery aid 6 and the drug delivery device 1 may be part of a drug delivery kit, comprising one or more drug delivery aids 6 and at least one drug delivery device 1. In particular, a drug delivery kit may comprise one drug delivery aid 6 and more than one drug delivery devices 1.

In another exemplary embodiment the retaining element 14 may have a different shape, e.g. as a clip arranged to be snap fitted on the housing 2 of the drug delivery device 1.

The term "drug" or "medicament", as used herein, means a pharmaceutical formulation containing at least one pharmaceutically active compound, wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
   H-(Lys)5-des Pro36, des Pro37 Exendin-4(1-39)-NH2,
   des Pro36 Exendin-4(1-39),
   des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(02)25, Asp28] Exendin-4(1-39),
   des Pro36 [Trp(02)25, IsoAsp28] Exendin-4(1-39),
   des Pro36 [Met(O)14 Trp(02)25, Asp28] Exendin-4(1-39),
   des Pro36 [Met(O)14 Trp(02)25, IsoAsp28] Exendin-4(1-39); or
des Pro36 [Asp28] Exendin-4(1-39),
   des Pro36 [IsoAsp28] Exendin-4(1-39),
   des Pro36 [Met(O)14, Asp28] Exendin-4(1-39),
   des Pro36 [Met(O)14, IsoAsp28] Exendin-4(1-39),
   des Pro36 [Trp(02)25, Asp28] Exendin-4(1-39),
   des Pro36 [Trp(02)25, IsoAsp28] Exendin-4(1-39),
   des Pro36 [Met(O)14 Trp(02)25, Asp28] Exendin-4(1-39),
   des Pro36 [Met(O)14 Trp(02)25, IsoAsp28] Exendin-4(1-39),
wherein the group -Lys6-NH2 may be bound to the C-terminus of the Exendin-4 derivative;
or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(02)25, Asp28] Exendin-4(1-39)-Lys6-NH2,
H-des Asp28 Pro36, Pro37, Pro38 [Trp(02)25] Exendin-4(1-39)-NH2,
H-(Lys)6-des Pro36, Pro37, Pro38 [Trp(02)25, Asp28] Exendin-4(1-39)-NH2,
H-Asn-(Glu)5-des Pro36, Pro37, Pro38 [Trp(02)25, Asp28] Exendin-4(1-39)-NH2,
des Pro36, Pro37, Pro38 [Trp(02)25, Asp28] Exendin-4(1-39)-(Lys)6-NH2,
H-(Lys)6-des Pro36, Pro37, Pro38 [Trp(02)25, Asp28] Exendin-4(1-39)-(Lys)6-NH2,
H-Asn-(Glu)5-des Pro36, Pro37, Pro38 [Trp(02)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(02)25, Asp28] Exendin-4(1-39)-Lys6-NH2,
H-des Asp28 Pro36, Pro37, Pro38 [Met(O)14, Trp(02)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(02)25, Asp28] Exendin-4(1-39)-NH2,
des Pro36, Pro37, Pro38 [Met(O)14, Trp(02)25, Asp28] Exendin-4(1-39)-(Lys)6-NH2,
H-(Lys)6-des Pro36, Pro37, Pro38 [Met(O)14, Trp(02)25, Asp28] Exendin-4(S1-39)-(Lys)6-NH2,
H-Asn-(Glu)5-des Pro36, Pro37, Pro38 [Met(O)14, Trp(02)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (∼150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCl or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

### List of References

- 1: drug delivery device
- 2: housing
- 3: needle cover
- 4: injection needle
- 5: trigger button
- 6: drug delivery aid
- 7: contact element
- 8: first surface
- 9: first aperture
- 10: recess
- 11: handle
- 12: second surface
- 13: surface structure
- 14: retaining element
- 15: second aperture
- D: distal direction
- P: proximal direction

## Claims

1. Drug delivery aid (6), comprising a substantially flat contact element (7) with a first surface (8) adapted to support a drug delivery device (1) with an extendable injection needle (4), and a second surface (12) opposite the first surface (8) adapted to be placed on an injection site, wherein a first aperture (9) is arranged in the contact element (7) adapted to allow extension of the injection needle (4) of the drug delivery device (1) through the first aperture (9).

2. Drug delivery aid (6) according to claim 1, wherein the contact element (7) is substantially disk shaped.

3. Drug delivery aid (6) according to claim 2, wherein the first surface (8) and the second surface (12) are substantially circular.

4. Drug delivery aid (6) according to claim 2, wherein the first surface (8) and the second surface (12) are substantially elliptical.

5. Drug delivery aid (6) according to one of the preceding claims, wherein a recess (10) is arranged around the first aperture (9) for centering a distal end of a drug delivery device (1) such that the injection needle (4) is axially aligned with the first aperture (9).

6. Drug delivery aid (6) according to one of the preceding claims, wherein a handle (11) is arranged on the contact element (7).

7. Drug delivery aid (6) according to one of the preceding claims, wherein one end of the handle (11) is arranged on the contact element (7), wherein another end of the handle (11) is attached to a retaining element (14) for releasably attaching the drug delivery aid (6) to a drug delivery device (1).

8. Drug delivery aid (6) according to one of the claims 6 or 7, wherein at least the handle (11) and/or the retaining element (14) are/is resilient.

9. Drug delivery aid (6) according to one of the claims 7 or 8, wherein the retaining element (14) comprises a second aperture (15) for allowing insertion of the drug delivery device (1) through the second aperture (15).

10. Drug delivery aid (6) according to claim 9, wherein an internal diameter of the second aperture (15) is smaller than an external diameter of the drug delivery device (1).

11. Drug delivery aid (6) according to one of the preceding claims, wherein the second surface (12) comprises a surface structure (13).

12. Drug delivery aid (6) according to claim 11, wherein the surface structure (13) comprises a plurality of pins.

13. Drug delivery aid (6) according to claim 12, wherein the pins comprise a mushroom shape.

14. Drug delivery kit, comprising a drug delivery aid (6) according to one of the preceding claims and at least one drug delivery device (1).

15. Drug delivery kit according to claim 14, wherein the drug delivery device (1) comprises a housing (2) and a needle cover (3) telescoped in a distal end of the housing (2) and adapted to be depressed towards the housing (2) for triggering an injection or for unlocking a trigger button (5) on the housing (2), wherein the recess (10) in the drug delivery aid (6) has a diameter corresponding to a diameter of the needle cover (3).
